# FASCICULE DE BREVET EUROPEEN

(11) **EP 2 529 910 B1**
(45) Date de publication et mention de la délivrance du brevet: **27.07.2016**
(21) Numéro de dépôt: 12358003.7
(22) Date de dépôt: 29.05.2012
(51) Int. Cl.: B29C 33/38, B23C 3/16, B25J 18/04, A61F 2/50

(54) **Procédé et dispositif d'usinage d'un moule pour la fabrication d'un appareil orthopédique**
Verfahren und Vorrichtung zur Bearbeitung einer Form für die Herstellung eines orthopädischen Geräts
Method and device for machining a mould for manufacturing an orthopaedic appliance

(30) Priorité: 30.05.2011 FR 1101660
(43) Date de publication de la demande: 05.12.2012
(73) Titulaire: Orthopedie Bontoux G.A., 84000 Avignon (FR)
(72) Inventeur: Paitel, Yann, 84130 Le Pontet (FR)
(74) Mandataire: Marchand, André

(56) Documents cités:
- DE-A1-102005 001 600
- US-A1- 2009 302 496
- US-A1- 2011 115 791
- US-B1- 6 865 442
- Delcam plc, AMS Division: "PowerMILL Robot Interface; 2011 - What's New?", FTP Listing of /pdf/powermill/en/ at arrow.delcam.com , 28 février 2011 (2011-02-28), XP002682441, Extrait de l'Internet: URL:ftp://arrow.delcam.com/pdf/powermill/e n/ [extrait le 2012-08-27]
- Mark Albert: "An Overview Of 3 + 2 Machining", Modern Machine Shop , 4 octobre 2006 (2006-10-04), XP002667479, Extrait de l'Internet: URL:http://www.mmsonline.com/articles/an-o verview-of-3-2-machining [extrait le 2012-01-19]
- Makino: "2+3 Machining:A faster, higher accuracy 5-axis machining technique", Knol, 27 mars 2009 (2009-03-27), XP002667478, Extrait de l'Internet: URL:http://knol.google.com/k/2-3-machining # [extrait le 2012-01-19]

## Description

### DOMAINE TECHNIQUE

La présente invention est relative à un procédé d'usinage d'une pièce telle qu'un moule pour la fabrication d'un appareil orthopédique, et à un dispositif d'usinage d'une telle pièce.

Le domaine technique de l'invention est celui de la fabrication d'appareils - ou appareillages - orthopédiques.

L'invention s'applique notamment à la fabrication d'orthèses et prothèses externes, telles qu'un corset, une genouillère, une orthèse cruro-pédieuse, une orthèse suro-pédieuse, un corset siège, ou une prothèse du membre inférieur, par exemple.

### ETAT DE LA TECHNIQUE

Pour la fabrication de tels appareils orthopédiques, on fabrique généralement un moule sur lequel une partie au moins d'un appareil orthopédique est réalisée par thermoformage ou par stratification (en utilisant une résine thermodurcissable).

Pour que cet appareil présente une forme et des dimensions adaptées à la morphologie d'un patient déterminé, on prend des mesures de la partie du corps du patient qui doit recevoir l'appareil orthopédique, puis on détermine la forme et les dimensions du moule à réaliser, en fonction des mesures prises. On peut notamment déterminer la forme tridimensionnelle du moule à partir d'une numérisation en trois dimensions de la forme de la partie du corps à appareiller, ou bien en adaptant une forme paramétrique en fonction de mesures propres au patient, à l'aide d'un logiciel de conception (CAO).

Le moule peut être réalisé par usinage, i.e. par enlèvement de matière, à partir d'un bloc de mousse de matière plastique (polyuréthane ou polyéthylène notamment).

Comme indiqué dans le brevet FR2885518, cet usinage peut être fait à l'aide d'une fraiseuse.

L'usinage à l'aide d'une fraiseuse à trois axes ne permet pas d'obtenir un moule présentant une contre dépouille, ce qui est fréquent pour les moules de fabrication d'orthèses et prothèses externes.

L'utilisation d'une fraiseuse est limitée à l'usinage de moules présentant une forme apparentée à celle d'un cylindre ou d'une pyramide, ce qui n'est pas le cas de moules pour différents types d'orthèses et prothèses externes.

Cette limitation peut parfois être surmontée en utilisant une fraiseuse à cinq axes, mais la taille et le coût d'une fraiseuse augmente fortement avec la taille de la pièce à usiner.

Il en est de même avec une machine à cinq ou six axes telle que celle mentionnée dans la demande de brevet US2011/115791.

Le document Mark Albert, « An Overview Of 3 + 2 Machining », Modern Machine Shop, 4 octobre 2006, décrit un procédé d'usinage dans lequel un outil de coupe d'une machine d'usinage à cinq axes est bloqué dans une position inclinée en utilisant deux axes rotatifs de la machine.

Le document MAkino, « 2 + 3 Machining : A faster, higher accuracy 5-axis machining technique », Knol, 27 mars 2009, décrit un procédé d'usinage dans lequel le positionnement d'axes A et C rotatifs secondaires - qui sont les axes les moins précis - est suivi de l'usinage avec les axes X, Y, et Z plus précis.

### EXPOSÉ DE L'INVENTION

Les tentatives d'usinage d'un moule pour la fabrication d'un appareil orthopédique à l'aide d'un robot portant un outil (tel qu'une fraise) par l'intermédiaire d'une broche servant à entrainer l'outil en rotation, et d'un plateau rotatif synchronisé avec les axes du robot et sur lequel est fixé une ébauche, n'ont à ce jour pas donné satisfaction.

La commande de certains robots d'usinage nécessite une phase préalable d'apprentissage pour chaque géométrie de pièce à usiner, notamment pour éviter des collisions entre la structure du robot - et l'outil qu'il porte -, et la pièce usinée ou l'ébauche en cours d'usinage.

Pour la fabrication d'un type déterminé d'appareil orthopédique - par exemple un corset - pour deux patients distincts, l'usinage par un robot des deux moules respectivement adaptés aux formes et dimensions des corps des deux patients distincts, nécessiterait d'effectuer deux étapes d'apprentissage, ce qui serait long et couteux.

Par ailleurs, des essais ont montré que le temps de fraisage d'un tel moule à l'aide d'un plateau rotatif et d'un robot à cinq ou six axes comportant cinq ou six articulations en série, est élevé.

Un objectif de l'invention est de proposer un procédé d'usinage d'une pièce « sur mesure » de forme complexe, en particulier un procédé d'usinage d'un moule pour la fabrication d'un appareil orthopédique, à l'aide d'un support rotatif et d'un robot à cinq axes au moins, qui soit rapide et facile à mettre en oeuvre, ainsi qu'un dispositif pour la mise en oeuvre de ce procédé.

Un objectif de l'invention est de proposer un procédé d'usinage d'une pièce unique de forme complexe, en particulier un procédé d'usinage d'un moule pour la fabrication d'un appareil orthopédique, à l'aide d'un support rotatif et d'un robot portant un outil rotatif et comportant au moins cinq articulations en série, ainsi qu'un dispositif pour la mise en oeuvre de ce procédé, qui soient améliorés et/ou qui remédient, en partie au moins, aux lacunes ou inconvénients des procédés et dispositifs d'usinage connus.

L'invention propose un procédé d'usinage selon la revendication 1. Différents aspects de l'invention sont définis dans les revendications dépendantes.

Selon un aspect de l'invention, il est proposé un procédé d'usinage dans lequel on dispose successivement une ébauche d'une pièce à usiner dans plusieurs positions (relatives) d'usinage distinctes par rapport au robot portant l'outil, le nombre de ces positions relatives distinctes étant réduit, et pour chacune de ces positions d'usinage, on usine l'ébauche en maintenant la direction de l'outil sensiblement fixe, en déplaçant l'extrémité de l'outil le long de plusieurs trajectoires successives qui s'étendent dans des plans de déplacement sensiblement parallèles, en particulier dans des plans parallèles à un axe de plus grande dimension de la pièce à usiner.

Selon un autre aspect de l'invention, il est proposé un procédé d'usinage dans lequel :
- a1) on fixe une ébauche de la pièce à usiner sur le support rotatif ;
- b1) en maintenant immobile le support rotatif, on usine partiellement l'ébauche à l'aide de l'outil rotatif porté par le robot, en maintenant l'axe (de rotation) de l'outil sensiblement parallèle à une direction déterminée par rapport à l'ébauche ;
- c1) on éloigne l'outil de l'ébauche partiellement usinée à l'issue de l'opération b1) ;
- d1) on choisit une seconde direction déterminée par rapport à l'ébauche différente de la première direction déterminée par rapport à l'ébauche, et le cas échéant on provoque une rotation du support rotatif selon un angle de rotation qui est égal à - ou déterminé en fonction de - l'angle formé par les première et seconde directions déterminées par rapport à l'ébauche; puis
- on répète l'opération b1) en maintenant l'axe de rotation de l'outil sensiblement parallèle à la seconde direction déterminée par rapport à l'ébauche.

En d'autres termes et selon un autre aspect de l'invention, il est proposé un procédé d'usinage d'une pièce à l'aide d'un support d'ébauche monté rotatif selon un axe de rotation, et d'un robot (à au moins cinq axes) comportant au moins cinq articulations rotoïdes en série, le robot comportant un actionneur rotatif associé à chacune des articulations et portant une broche pour entraîner en rotation un outil d'usinage tel qu'une fraise, dans lequel :
- a2) on fixe une ébauche de la pièce à usiner sur le support rotatif ;
- b2) en maintenant immobile le support rotatif de façon à disposer l'ébauche dans l'une desdites positions d'usinage, on entraîne l'outil en rotation et on commande les actionneurs rotatifs du robot pour déplacer l'outil interférant avec l'ébauche pour provoquer un enlèvement de matière, en maintenant sensiblement fixe l'orientation de l'axe de rotation de l'outil par rapport à un plan d'usinage, les actionneurs du robot étant commandés pour déplacer l'outil le long de plans de déplacement qui sont sensiblement parallèles à l'axe de rotation du support rotatif, sensiblement régulièrement espacés, et sensiblement perpendiculaires au plan d'usinage;
- c2) on éloigne l'outil de l'ébauche partiellement usinée à l'issue de l'opération b2) ;
- d2) on provoque une rotation du support rotatif pour disposer l'ébauche dans une autre position d'usinage par rapport au robot; puis
- on répète au moins une fois l'opération b2) en modifiant à chaque fois le plan d'usinage.

L'outil est de préférence constitué d'une fraise présentant une partie coupante - également dite « active » - comportant une portion cylindrique de petit diamètre terminée par une portion hémisphérique, et dont les dents - ou plaquettes ou autres organes de coupe - sont réparties sur ces deux portions.

En maintenant (sensiblement) fixe l'orientation de l'outil, en particulier en maintenant sensiblement constant l'angle formé par l'axe longitudinal - et de rotation - de l'outil et l'un desdits plans d'usinage, pendant chaque séquence d'usinage de l'ébauche au cours de laquelle l'ébauche est maintenue immobile, donc sans tenir compte de l'orientation « locale » de la surface externe à obtenir pour la pièce (le moule) considéré(e), on favorise des mouvements monotones de certaines au moins des articulations du robot, ce qui a pour effet d'augmenter la vitesse moyenne de déplacement de l'outil par le robot, et par conséquent de diminuer le temps nécessaire à l'usinage d'une pièce.

Ceci est également favorisé par le fait que l'ébauche est immobile pendant les opérations d'usinage.

Par « mouvement monotone », on entend un déplacement d'au moins un des segments - ou éléments du corps - du robot, qui résulte d'une commande d'un des actionneurs rotatifs du robot, et au cours duquel le sens de rotation de l'actionneur n'est pas inversé.

A titre d'exemple, pour un robot à six axes - et actionneurs - rotatifs en série, dans lequel on repère ces axes - de même que les actionneurs rotatifs et les articulations correspondant à ces axes - de AX1 à AX6 en partant de la base du robot jusqu'à son organe terminal, le fait de dissocier l'orientation de l'outil de la normale (locale) de la surface à usiner, permet au robot d'avoir une information pour la commande de l'actionneur correspondant à l'axe AX5, et permet de diminuer l'amplitude de mouvement des axes AX1, AX4, et AX6, en privilégiant des mouvements monotones et/ou de grande amplitude sur les axes AX2, AX3, et AX5.

Ainsi avec des mouvements angulaires de grande amplitude pour ces axes AX2, AX3, et AX5, le robot peut accélérer de façon significative pour ces axes AX2, AX3, et AX5.

Cette augmentation de la vitesse moyenne d'usinage est encore favorisée en prévoyant que l'axe de rotation du support rotatif soit parallèle à l'axe AX1 d'articulation mutuelle des deux premiers segments du robot, et le cas échéant en disposant cet axe dans une partie périphérique (i.e. éloignée de l'axe AX1 du robot) du volume de travail du robot, i.e. du volume accessible par l'outil porté par le robot.

Cette augmentation de la vitesse moyenne d'usinage peut également être favorisée en prévoyant de disposer le plan du support rotatif recevant l'ébauche - et par conséquent la base de l'ébauche -, à une cote mesurée le long d'un axe parallèle à l'axe AX1 du robot, qui soit telle que la cote sur cet axe du centre de la pièce et/ ou de l'ébauche soit confondu avec - ou voisin de - la cote, sur le même axe, de l'axe AX2 d'articulation mutuelle des second et troisième segments du robot.

Dans le cas où l'axe AX1 du robot et l'axe de rotation du support rotatif sont verticaux, le plan du support rotatif peut alors être placé sous « l'altitude » de l'axe AX2 du robot, à une distance égale ou voisine de la demi hauteur de l'ébauche (et/ou de la pièce à usiner). Ceci permet également d'utiliser le plus petit robot possible pour usiner une pièce déterminée.

Notamment pour l'usinage d'un moule pour la fabrication d'un appareil orthopédique, le nombre de positions d'usinage distinctes, qui correspond dans certains cas au nombre de rotations du support rotatif augmenté d'une unité - et au nombre d'opérations b1) ou b2) -, est généralement situé dans une plage allant de 2 à 8, en particulier situé dans une plage allant de 2 ou 3 à 6.

Notamment lorsque ce nombre de positions est situé dans une plage allant de 2 à 4, l'angle de rotation du support rotatif peut être de 90 degrés ou de 180 degrés. Dans ces cas notamment, les plans d'usinage peuvent être orthogonaux ou parallèles entre eux.

A la différence d'un procédé dans lequel la rotation du support rotatif de l'ébauche serait continue et/ou concomitante à l'usinage et aux déplacements de l'outil par le robot, dans un procédé selon l'invention, il pourra être rendu nécessaire d'opérer un dégagement ou « repli » de l'outil afin d'éloigner l'outil de l'ébauche le temps de procéder à la rotation du support rotatif, afin d'éviter une éventuelle interférence entre l'ébauche et l'outil, la broche porte outil, et/ou le robot. Dans ce cas, étonnamment, il a pu être constaté que la perte de temps occasionnée par ces mouvements de repli, affecte de façon peu perceptible la vitesse moyenne d'usinage obtenue.

Pour chaque dégagement ou « repli », on peut provoquer un déplacement de l'outil à rebours le long d'une partie substantielle de la trajectoire d'outil réalisée en dernier lieu, en particulier le long d'une moitié de la trajectoire d'outil réalisée en dernier lieu, suivi d'un déplacement de l'outil selon une normale au plan d'usinage correspondant à l'opération d'usinage en cours, dans le sens d'un éloignement relatif de l'outil et du plan d'usinage, et sur une distance au moins égale à la demi longueur de la partie « active » de l'outil.

Ceci peut permettre d'éviter de procéder à un apprentissage d'un mouvement de repli pour chaque séquence d'usinage et pour chaque pièce à usiner.

Selon un autre aspect de l'invention, il est également proposé un dispositif configuré de façon à exécuter un procédé selon l'invention. Un tel dispositif d'usinage d'un moule pour la fabrication d'un appareil orthopédique est défini dans la revendication 15. Il comporte notamment un support d'ébauche monté rotatif selon un axe de rotation, un robot (à cinq ou six axes) comportant cinq ou six articulations rotoïdes en série et portant une broche pour entraîner en rotation un outil d'usinage tel qu'une fraise, ainsi qu'une unité de commande - telle qu'une unité électronique de commande à microprocesseur - agencée, en particulier programmée, pour commander le fonctionnement des actionneurs du robot et pour commander la rotation du support d'ébauche rotatif, de façon à exécuter les opérations d'un procédé selon l'invention.

Selon un mode de réalisation, les axes de rotation des actionneurs du robot correspondant aux axes repérés AX4, AX5 d'articulation mutuelle des quatrième, cinquième, et sixième segments du robot, et le cas échéant l'axe AX6, comme indiqué ci avant, coïncident - ou concourent - en un point.

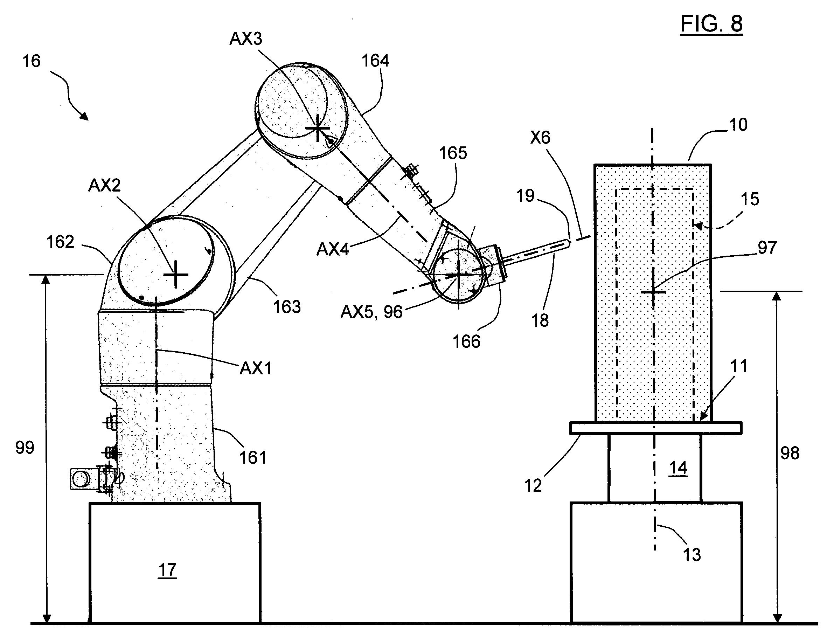

Notamment lorsque l'axe longitudinal (repéré X6 figure 8) de rotation de l'outil coïncide avec les axes AX4 et AX5 du robot, ce dernier peut comporter cinq axes seulement.

D'autres aspects, caractéristiques, et avantages de l'invention apparaissent dans la description suivante qui se réfère aux figures annexées et illustre, sans aucun caractère limitatif, des modes préférés de réalisation de l'invention.

### BREVE DESCRIPTION DES FIGURES

La figure 1 est une vue en perspective schématique illustrant des opérations d'un procédé de fabrication d'un moule pour la fabrication d'un corset.
La figure 2 est une vue en perspective schématique illustrant des opérations d'un procédé de fabrication de deux moules pour la fabrication de deux releveurs.
La figure 3 est une vue en perspective schématique illustrant des opérations d'un procédé de fabrication d'un moule pour la fabrication d'un corset-siège.
La figure 4 est une vue en perspective schématique illustrant des opérations d'un procédé de fabrication d'un moule pour la fabrication d'un verticaliseur (appareil permettant à un patient de prendre la position debout).

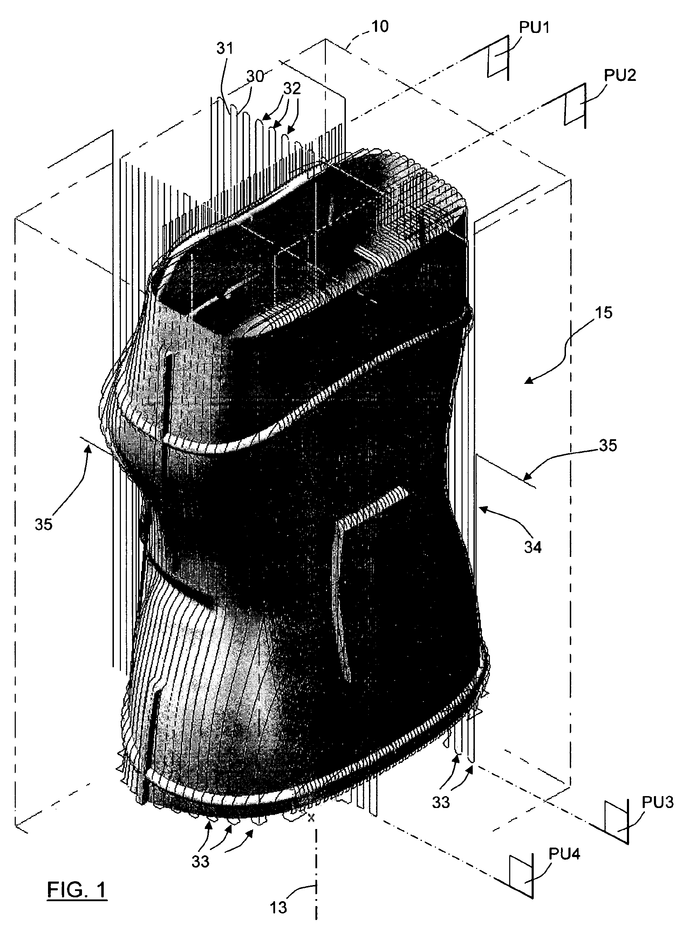

Sur les figures 1 à 4 sont essentiellement représentées les trajectoires de l'extrémité d'un outil de fraisage utilisé pour usiner un bloc de mousse de matière plastique.

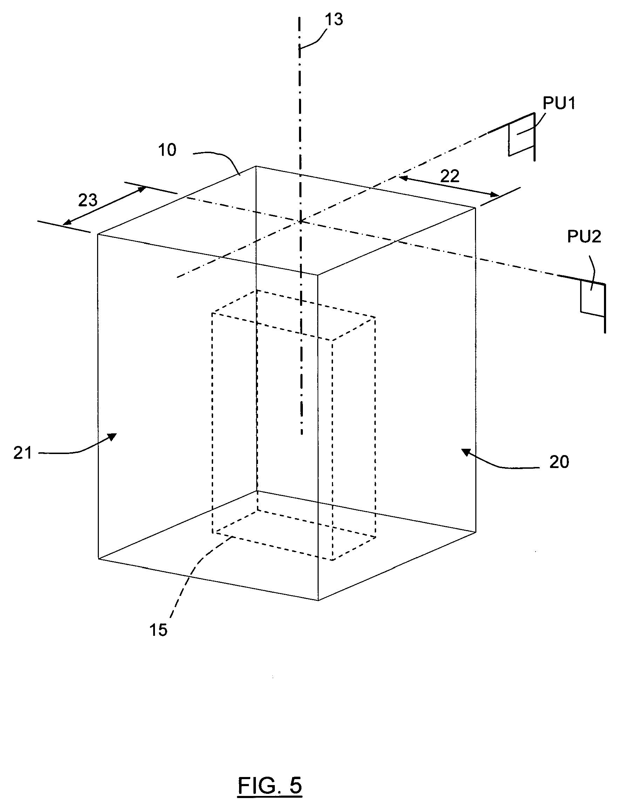

Les figures 5 à 7 sont des vues en perspective schématique illustrant des opérations successives d'un procédé de fabrication d'un moule pour la fabrication d'une pièce (fictive) en forme de parallélépipède rectangle.
La figure 8 est une vue illustrant schématiquement un robot et un support rotatif portant une ébauche de moule pour la fabrication d'un appareil orthopédique.

### DESCRIPTION DETAILLEE DE L'INVENTION

Sauf indication explicite ou implicite contraire, des éléments ou organes - structurellement ou fonctionnellement - identiques ou similaires sont désignés par des repères identiques sur les différentes figures.

Par référence à la figure 8 en particulier, pour la fabrication d'un moule 15 à partir d'une ébauche 10 constituée d'un bloc de mousse de matière plastique, on fixe l'ébauche par sa base 11 sur un support 12 qui est entrainé en rotation selon un axe 13 par un actionneur 14 tel qu'un moteur électrique.

Dans la configuration illustrée figure 8, le support 12 supportant l'ébauche 10 s'étend selon un plan horizontal ; l'axe 13 de rotation est vertical et sensiblement confondu avec les axes de symétrie et de plus grande dimension de l'ébauche 10 ainsi que du moule 15 à usiner, le moule à usiner et l'ébauche présentant chacun une forme de parallélépipède rectangle.

Le dispositif utilisé pour la fabrication du moule comporte en outre un robot 16 à cinq axes « série » fixé sur un socle 17 et utilisé pour déplacer un outil 18 de fraisage dont l'extrémité 19 est arrondie, en particulier hémisphérique.

Le corps du robot est essentiellement constitué de six segments 161 à 166 reliés entre eux deux à deux, « en série », par cinq articulations d'axes respectifs AX1 à AX5, chaque articulation comportant respectivement un actionneur rotatif pour assurer un déplacement relatif (en rotation) des deux segments successifs du robot que l'articulation considérée relie:
- le premier segment 161, qui est la base (fixe) du robot, est solidaire du socle 17 ;
- le second segment 162 est entrainé en rotation selon le premier axe AX1, par rapport au segment 161, par l'actionneur rotatif de la première articulation reliant le segment 162 au segment 161 ;
- le troisième segment 163 est entrainé en rotation selon le second axe AX2, par rapport au segment 162, par l'actionneur rotatif de la seconde articulation reliant le segment 163 au segment 162 ;
- le quatrième segment 164 est entrainé en rotation selon le troisième axe AX3, par rapport au segment 163, par l'actionneur rotatif de la seconde articulation reliant le segment 164 au segment 163 ;
- le cinquième segment 165 est entrainé en rotation selon le quatrième axe AX4, par rapport au segment 164, par l'actionneur rotatif de la seconde articulation reliant le segment 165 au segment 164;
- le sixième segment 166 est entrainé en rotation selon le cinquième axe AX5, par rapport au segment 165, par l'actionneur rotatif de la cinquième articulation reliant le segment 166 au segment 165.

L'outil 18, qui s'étend selon l'axe X6, est entrainé en rotation selon cet axe par une broche électrique montée sur le segment 166.

On peut observer figure 8 que le premier axe AX1 du robot est vertical, que les axes AX2 et AX3 sont horizontaux et parallèles, et que les axes AX4, AX5, et X6 présentent un point d'intersection commun.

Le dispositif d'usinage comporte en outre une unité électronique de commande à microprocesseur - non représentée -, telle qu'un ordinateur associé à une mémoire contenant des données représentatives du moule à usiner, qui est reliée aux actionneurs du robot et à l'actionneur du support rotatif. Cette unité de commande est programmée pour commander le fonctionnement des actionneurs du robot et pour commander la rotation du support d'ébauche rotatif, comme décrit dans la présente.

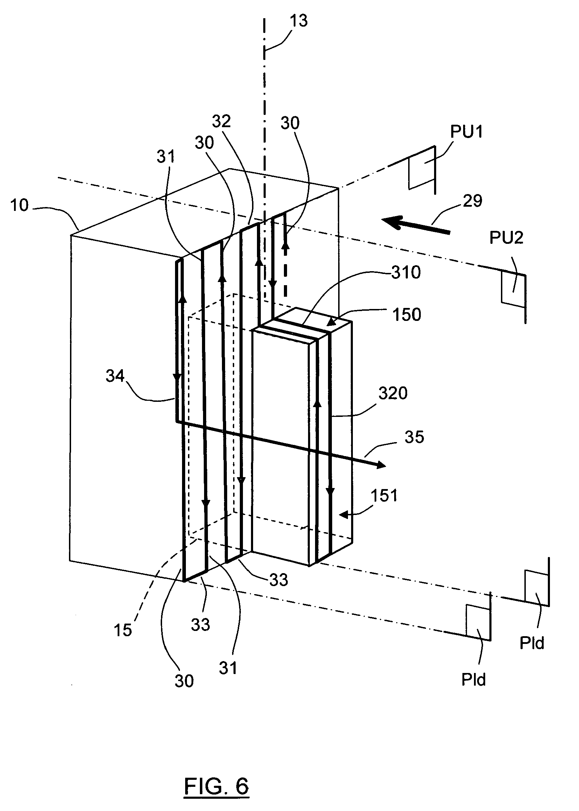

Par référence aux figures 5 à 8, l'usinage d'une pièce 15 de forme parallélépipédique dans l'ébauche 10, peut s'effectuer de la façon suivante :
- on choisit deux plans d'usinage PU 1, PU2 qui sont parallèles à - et contiennent, dans la configuration représentée figure 5 - l'axe 13 de plus grande dimension de la pièce 15 à usiner; ces plans sont choisis de telle sorte que leurs distances respectives 22, 23 aux faces externes 20, 21 de l'ébauche le long desquelles ces plans s'étendent respectivement, soient inférieures à la longueur « active » de l'outil de fraisage, de façon à éviter une interférence entre l'ébauche et la partie « inactive » de l'outil et/ou avec la broche portant l'outil ;
- on commande les actionneurs du robot de façon à positionner l'extrémité de l'outil de fraisage, puis à déplacer cette extrémité d'outil, de façon à ce que cette extrémité d'outil « balaye » une surface constituée par le raccordement de la surface externe 150, 151 d'une première partie de la pièce 15 qui s'étend entre le premier plan d'usinage PU1 et le robot, et la partie de ce plan PU1 qui s'étend à l'intérieur de l'ébauche et autour de cette surface externe d'une première partie de la pièce 15 ; à cet effet, comme illustré figure 6, on déplace l'extrémité de l'outil de fraisage selon une trajectoire qui comporte des tronçons 30 de trajectoire ascendante, des tronçons 31 de trajectoire descendante, et des tronçons 32, 33 de liaison reliant chacun un tronçon 30 de trajectoire ascendante au tronçon 31 de trajectoire descendante suivant (respectivement précédent).

Comme on peut l'observer figure 6 notamment, certains des tronçons 30, 31 de trajectoire sont rectilignes, parallèles, et contenus dans le plan PU1, tandis que d'autres tronçons 30, 31 de trajectoire comportent des portions - telles que celles repérées 310 et 320 - qui s'étendent à distance du plan PU1, ces portions 310, 320 de trajectoire s'étendant sur des portions 150, 151 de la surface externe de la pièce 15 à usiner.

Généralement, pendant que l'extrémité de l'outil est déplacée par le robot selon ces trajectoires 30 à 33, l'axe (X6 figure 8) longitudinal - et de rotation - de l'outil est maintenu parallèle à une direction 29 peu inclinée par rapport à la normale au plan PU 1 : l'inclinaison de l'axe X6 par rapport à cette normale est généralement inférieure à - ou voisine de - 20 ou 30 degrés.

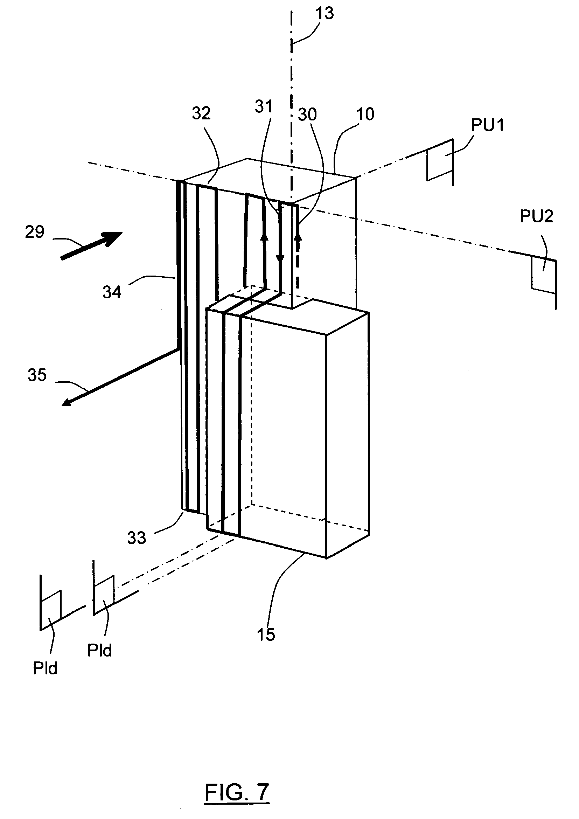

Suite à cet usinage partiel de l'ébauche 10 illustré figure 6, on modifie l'orientation mutuelle de l'ébauche et de l'outil de fraisage, généralement en modifiant l'orientation mutuelle de l'ébauche et du robot, à l'aide du support rotatif d'indexation de l'ébauche ; on provoque une rotation du plateau et de l'ébauche, selon l'axe 13, de 90 degrés dans cet exemple, pour ensuite procéder aux opérations d'usinage par rapport au second plan d'usinage PU2 qui est perpendiculaire au plan PU1, comme illustré figure 7.

Préalablement à cette rotation de l'ébauche, on fait généralement suivre à l'outil une trajectoire de dégagement qui comporte un tronçon 34 qui s'étend le long du tronçon 31 de trajectoire parcouru en dernier lieu. Ces tronçons 34, 31 sont sensiblement confondus, la longueur du tronçon 34 étant voisine de la demi-longueur du tronçon 31 réalisé en dernier lieu.

La trajectoire de dégagement comporte en outre un tronçon 35 de trajectoire, ce tronçon s'étendant sensiblement perpendiculairement au plan PU 1 ; la longueur de ce tronçon est au mois égale à celle de la partie active de l'outil 18, par exemple au moins égale à la longueur de cet outil.

Après la rotation de l'ébauche, on commande les actionneurs du robot de façon à positionner l'extrémité de l'outil de fraisage, puis à déplacer cette extrémité d'outil, de façon à ce que cette extrémité d'outil se déplace le long d'une surface constituée par le raccordement de la surface externe d'une seconde partie de la pièce 15 qui s'étend entre le second plan d'usinage PU2 et le robot, et la partie de ce plan PU2 qui s'étend à l'intérieur de l'ébauche restante et autour de cette surface externe d'une seconde partie de la pièce 15.

A cet effet, comme décrit précédemment et comme illustré figure 7, on déplace l'extrémité de l'outil de fraisage selon une trajectoire qui comporte des tronçons 30, 31 de trajectoire s'étendant dans des plans de déplacement Pld sensiblement perpendiculaires au plan PU2, des tronçons 32, 33 de liaison, et en dernier lieu des tronçons 34, 35 de trajectoire de dégagement de l'outil.

Les plans Pld de déplacement dans lesquels s'étendent les tronçons 30, 31 de trajectoire le long desquels la vitesse de déplacement de l'outil est maximale, sont généralement régulièrement espacés d'une distance qui peut être voisine du quart - ou du huitième - du diamètre de l'outil de fraisage.

A titre d'exemple, pour une fraise ayant un diamètre de 20 millimètres, le pas d'espacement de ces plans peut être de l'ordre de 2,5 à 5 millimètres environ.

Pour l'usinage final de la pièce 15, postérieurement à la configuration d'usinage partiel illustrée figure 7, on modifie une seconde fois l'orientation mutuelle de l'ébauche et du robot, par une rotation de 90 degrés du plateau supportant l'ébauche, pour procéder aux opérations finales de fraisage, par référence au premier plan PU1 d'usinage.

On maintient alors l'axe de l'outil de fraisage selon une direction opposée à celle repérée 29 figure 6, et on procède à l'usinage final en déplaçant l'outil tenu par le robot selon une trajectoire similaire à celles illustrées figures 6 et 7.

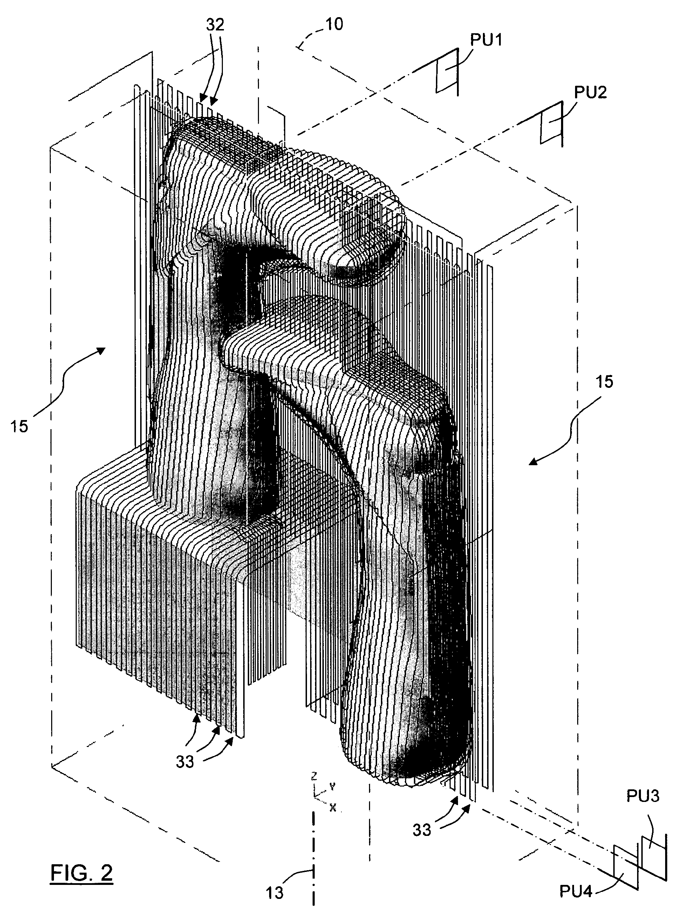

Pour les modes de réalisation illustrés figures 1 et 2, on procède de la façon décrite précédemment. On peut observer sur ces figures que l'usinage est réalisé selon quatre plans d'usinage PU1 à PU4 qui sont parallèles deux à deux, et parallèles à l'axe 13.

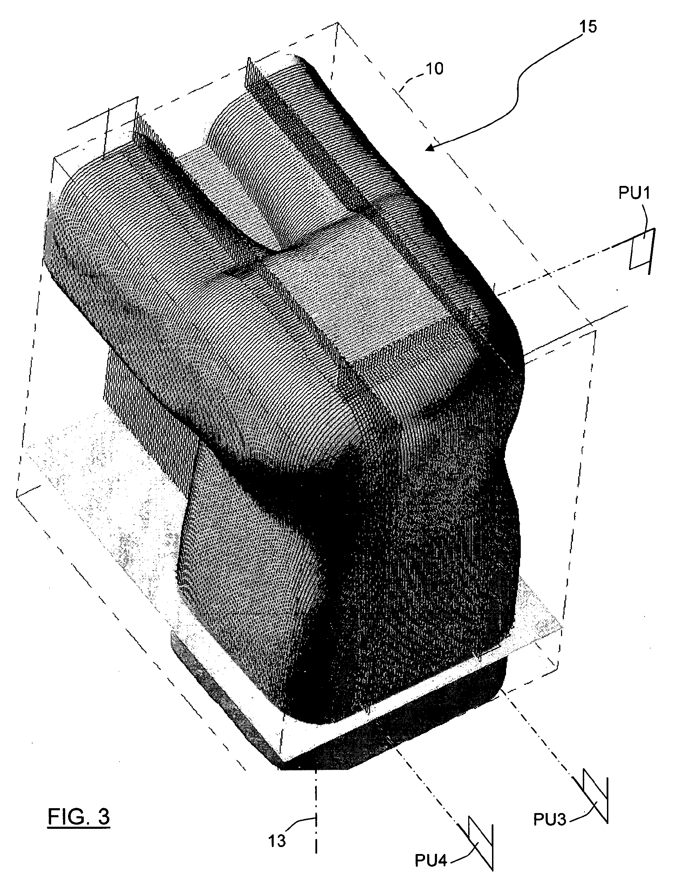

On procède de façon similaire pour le mode de réalisation illustré figure 3, en orientant et en déplaçant l'outil de fraisage par référence à trois plans PU1, PU3, PU4 qui sont parallèles à l'axe 13, selon trois séquences respectives d'usinage. En outre, dans une quatrième séquence d'usinage, on provoque le déplacement de l'outil par référence à un quatrième plan d'usinage (non représenté) qui est orthogonal à l'axe 13.

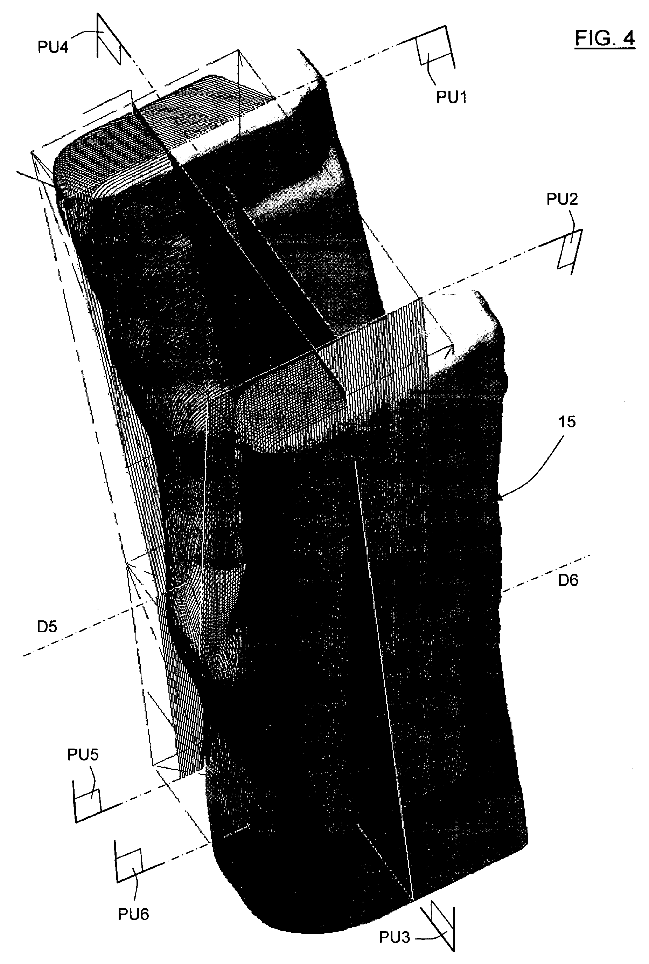

On procède également de façon similaire pour le mode de réalisation illustré figure 4, en déplaçant l'outil par référence à six plans PU1 à PU6 d'usinage, en six séquences d'usinage.

Dans ce mode de réalisation, les plans PU3 à PU6 sont parallèles deux à deux et parallèles à l'axe 13, tandis que les plans PU1 et PU2 - qui coupent respectivement les plans PU5 et PU6 selon les droites D5 et D6 - sont inclinés par rapport à l'axe 13.

Dans le même but qu'indiqué précédemment, les plans PU1 et PU2 s'étendent respectivement le long des deux axes de plus grande dimension des parties du moule à usiner qui correspondent aux cuisses d'un patient. Dans ce mode de réalisation, l'angle formé par les plans PU1 et PU2 est déterminé par l'abduction des jambes du patient.

Par ailleurs, le fait d'usiner chaque partie latérale externe (gauche et droite) du moule selon deux séquences d'usinage, respectivement par référence à deux plans d'usinage mutuellement inclinés, i.e. les plans PU1 et PU5 pour la partie gauche du moule et les plans PU2 et PU6 pour la partie droite du moule, permet de limiter les amplitudes de mouvement d'au moins un des axes du robot, en particulier de l'axe AX5.

L'invention tire profit du fait que l'architecture électromécanique d'un robot lui procure une grande capacité d'accélération pour peu que l'on commande les actionneurs du robot de façon à faire suivre à l'outil des trajectoires « simples » (ou « uniformes »), idéalement des trajectoires circulaires, rectilignes, ou des trajectoires caractérisables par une équation simple.

Contrairement à une fraiseuse qui déplace l'outil à une vitesse de déplacement constante, le robot accélère et décélère entre chaque point caractérisant une inflexion de la trajectoire à suivre ; ainsi, plus la distance entre deux points d'inflexion est grande, plus le robot a la possibilité d'atteindre sa vitesse maximum.

L'invention tire également profit du fait que les inversions de sens de rotation des motoréducteurs des articulations du robot sont moins fréquentes - sinon éliminées - lorsque l'outil décrit les trajectoires décrites précédemment, en particulier lorsque l'outil décrit les portions de trajectoire 30, 31, ce qui permet d'augmenter la vitesse moyenne de déplacement de l'outil par le robot.

## Revendications

1. Procédé d'usinage d'un moule (15) pour la fabrication d'un appareil orthopédique, à l'aide d'une machine (16) à au moins cinq axes portant un outil (18) rotatif, dans lequel la machine est un robot qui comporte au moins cinq articulations (AX1 à AX5) rotoïdes en série, et dans lequel :
- on fixe une ébauche (10) du moule à un support (12) monté rotatif selon un axe (13) de rotation,
- on dispose successivement une ébauche (10) du moule dans plusieurs positions d'usinage distinctes par rapport au robot, par rotation du support rotatif, et
- pour chacune de ces positions d'usinage, on usine l'ébauche en maintenant la direction (29) de l'outil sensiblement fixe, et en déplaçant l'extrémité de l'outil le long de trajectoires (30, 31) successives qui s'étendent dans des plans (Pld) de déplacement parallèles.

2. Procédé selon la revendication 1 dans lequel les plans (Pld) de déplacement sont parallèles à un axe (13) de plus grande dimension du moule.

3. Procédé selon la revendication 1 ou 2 dans lequel les plans (Pld) de déplacement sont espacés d'un pas voisin du quart ou du huitième du diamètre de la partie coupante de l'outil (18).

4. Procédé selon l'une des revendications 1 à 3 qui comporte les opérations suivantes :
- a1) fixer l'ébauche du moule sur le support (12) rotatif ;
- b1) en maintenant immobile le support rotatif, usiner partiellement l'ébauche à l'aide de l'outil rotatif en maintenant l'axe (X6) (de rotation) de l'outil sensiblement parallèle à une direction (29) déterminée par rapport à l'ébauche ;
- c1) éloigner (35) l'outil de l'ébauche partiellement usinée à l'issue de l'opération b1) ;
- d1) choisir une seconde direction déterminée par rapport à l'ébauche différente de la première direction déterminée par rapport à l'ébauche, et le cas échéant provoquer une rotation du support rotatif selon un angle de rotation qui est égal à - ou déterminé en fonction de - l'angle formé par la première et la seconde direction déterminée par rapport à l'ébauche; puis
- répéter l'opération b1) en maintenant l'axe de rotation de l'outil sensiblement parallèle à la seconde direction déterminée par rapport à l'ébauche.

5. Procédé selon l'une des revendications 1 à 4, le robot comportant un actionneur rotatif associé à chacune des articulations et portant une broche pour entraîner en rotation l'outil d'usinage, dans lequel :
- a2) on fixe l'ébauche du moule sur le support rotatif ;
- b2) en maintenant immobile le support rotatif de façon à disposer l'ébauche dans l'une desdites positions d'usinage, on entraîne l'outil en rotation et on commande les actionneurs rotatifs du robot pour déplacer l'outil interférant avec l'ébauche afin de provoquer un enlèvement de matière, en maintenant sensiblement fixe l'orientation de l'axe de rotation de l'outil par rapport à un plan d'usinage (PU1 à PU6), les actionneurs du robot étant commandés pour déplacer l'outil le long des plans de déplacement qui sont sensiblement parallèles à l'axe de rotation du support rotatif, sensiblement régulièrement espacés, et sensiblement perpendiculaires au plan d'usinage;
- c2) on éloigne l'outil de l'ébauche partiellement usinée à l'issue de l'opération b2) ;
- d2) on provoque une rotation du support rotatif pour disposer l'ébauche dans une autre position d'usinage par rapport au robot; et
- on répète au moins une fois l'opération b2) en modifiant le plan d'usinage.

6. Procédé selon l'une des revendications 1 à 5 dans lequel l'outil est constitué d'une fraise présentant une partie active comportant une portion cylindrique et/ou une portion (19) hémisphérique, et des organes de coupe répartis sur cette ou ces portion(s).

7. Procédé selon l'une des revendications 1 à 6 dans lequel l'axe (13) de rotation du support rotatif est parallèle à l'axe (AX1) d'articulation des deux premiers segments (161, 162) du robot et s'étend dans une partie périphérique du volume de travail du robot.

8. Procédé selon l'une des revendications 1 à 7 dans lequel le plan du support rotatif recevant l'ébauche est disposé à une cote mesurée le long d'un axe parallèle à l'axe (AX1) d'articulation mutuelle des deux premiers segments (161, 162) du robot, qui est telle que la cote (98) sur cet axe du centre (97) du moule ou de l'ébauche soit confondu avec - ou voisin de - la cote (99), sur le même axe, de l'axe (AX2) d'articulation mutuelle des second et troisième segments (162, 163) du robot.

9. Procédé selon l'une des revendications 1 à 8 dans lequel l'axe (AX1) d'articulation mutuelle des deux premiers segments (161, 162) du robot et l'axe (13) de rotation du support rotatif sont verticaux, le plan du support rotatif étant placé sous l'altitude de l'axe (AX2) d'articulation mutuelle des second et troisième segments (162, 163) du robot, à une distance égale ou voisine de la demi hauteur de l'ébauche ou du moule.

10. Procédé selon l'une des revendications 1 à 9 dans lequel le nombre de positions d'usinage distinctes, qui correspond le cas échéant au nombre de rotations du support rotatif - et au nombre d'opérations b1) ou b2) - augmenté d'une unité, est situé dans une plage allant de 2 à 8, en particulier situé dans une plage allant de 2 à 6.

11. Procédé selon l'une des revendications 4 à 10 dans lequel l'angle de rotation du support rotatif est de 90 degrés ou de 180 degrés.

12. Procédé selon l'une des revendications 5 à 11 dans lequel au moins deux des plans d'usinage (PU1 à PU6) sont orthogonaux.

13. Procédé selon l'une des revendications 5 à 12 dans lequel au moins deux des plans d'usinage (PU1 à PU6) sont parallèles.

14. Procédé selon l'une des revendications 4 à 13 dans lequel, pour chaque opération c1) ou c2) de dégagement ou « repli », on provoque un déplacement (34) de l'outil à rebours le long d'une moitié de la trajectoire d'outil réalisée en dernier lieu, suivi d'un déplacement (35) de l'outil selon une normale au plan d'usinage (PU 1 à PU6) correspondant à l'opération d'usinage en cours, dans le sens d'un éloignement relatif de l'outil et du plan d'usinage, et sur une distance au moins égale à la demi longueur de la partie « active » de l'outil.

15. Dispositif d'usinage d'un moule (15) pour la fabrication d'un appareil orthopédique, le dispositif comportant un support (12) d'ébauche (10) monté rotatif selon un axe (13) de rotation, un robot (16) comportant au moins cinq articulations (AX1 à AX5) rotoïdes en série et portant une broche pour entraîner en rotation un outil (18) d'usinage, ainsi qu'une unité de commande - telle qu'une unité électronique de commande à microprocesseur - programmée pour commander le fonctionnement des actionneurs du robot et pour commander la rotation du support d'ébauche rotatif, de façon à exécuter les opérations d'un procédé selon l'une des revendications 1 à 14.

16. Dispositif selon la revendication 15 dans lequel les axes (AX4, AX5) d'articulation mutuelle des quatrième, cinquième, et sixième segments (164, 165, 166) du robot, et le cas échéant l'axe (X6) de rotation de l'outil (18), concourent en un point (96).

## Patentansprüche

1. Verfahren zur Bearbeitung einer Form (15) zur Herstellung eines orthopädischen Apparates mit Hilfe einer Maschine (16) mit mindestens fünf Achsen, die ein rotierendes Werkzeug (18) trägt, in dem die Maschine ein Roboter ist, der über mindestens fünf Drehgelenke (AX1 bis AX5) in Reihe verfügt, und in dem
- ein Rohling (10) der Form auf einem Träger (12) befestigt wird, der um eine Drehachse (13) drehbar montiert ist,
- ein Rohling (10) der Form nacheinander durch Drehung des drehbaren Trägers in mehrere verschiedene Bearbeitungsstellungen relativ zum Roboter gebracht wird, und
- in jeder dieser Bearbeitungsstellungen der Rohling bearbeitet wird, wobei die Richtung (29) des Werkzeugs im Wesentlichen beibehalten wird und indem das Ende des Werkzeugs längs aufeinander folgender Bahnen (30, 31) bewegt wird, die in parallelen Bewegungsebenen (Pld) verlaufen.

2. Verfahren nach Patentanspruch 1, in dem die Bewegungsebenen (Pld) parallel zu einer Achse (13) der größten Abmessung der Form sind.

3. Verfahren nach Patentanspruch 1 oder 2, in dem die Bewegungsebenen (Pld) um einen Schritt nahe dem Viertel oder Achtel des Durchmessers des schneidenden Teils des Werkzeugs (18) beabstandet sind.

4. Verfahren nach einem der Patentansprüche 1 bis 3, das die folgenden Schritte umfasst:
- a1) Befestigen des Formrohlings auf dem drehbaren Träger (12),
- b1) teilweise Bearbeitung des Rohlings mit Hilfe des rotierenden Werkzeugs bei feststehend gehaltenem drehbarem Träger, wobei die (Rotations-)Achse (X6) des Werkzeugs im Wesentlichen parallel zu einer Richtung (29) gehalten wird, die relativ zum Rohling festgelegt ist,
- c1) Entfernen (35) des Werkzeugs vom teilweise bearbeiteten Rohling zum Abschluss des Arbeitsschrittes b1),
- d1) Auswahl einer zweiten relativ zum Rohling festgelegten Richtung, die von der ersten relativ zum Rohling festgelegten Richtung verschieden ist, und gegebenenfalls Veranlassen einer Drehung des drehbaren Trägers um einen Drehwinkel, der dem Winkel gleich ist - oder in Abhängigkeit von dem Winkel festgelegt ist -, den die erste mit der zweiten relativ zum Rohling festgelegten Richtung einschließt, und danach
- Wiederholung des Schrittes b1), wobei die Rotationsachse des Werkzeugs im Wesentlichen parallel zur zweiten relativ zum Rohling festgelegten Richtung gehalten wird.

5. Verfahren nach einem der Patentansprüche 1 bis 4, wobei der Roboter ein Rotationswirkglied umfasst, das mit jedem der Gelenke verbunden ist und eine Welle trägt, um das Bearbeitungswerkzeug in Rotation zu versetzen, in dem
- a2) der Formrohling auf dem drehbaren Träger (12) befestigt wird,
- b2) bei feststehend gehaltenem drehbarem Träger, derart, dass der Rohling in einer der genannten Bearbeitungsstellungen angeordnet wird, das Werkzeug in Rotation versetzt wird und die Rotationswirkglieder des Roboters betätigt werden, um das Werkzeug zu bewegen, das auf den Rohling einwirkt, um ein Abheben von Material zu bewirken, wobei die Richtung der Rotationsachse des Werkzeugs relativ zur Bearbeitungsebene (PU1 bis PU6) im Wesentlichen beibehalten wird, wobei die Wirkglieder der Roboters derart gesteuert werden, dass das Werkzeug in den Bewegungsebenen bewegt wird, die zur Drehachse des drehbaren Trägers im Wesentlichen parallel sind, im Wesentlichen regelmäßig beabstandet sind und auf der Bearbeitungsebene im Wesentlichen senkrecht stehen,
- c2) zum Abschluss des Arbeitsschrittes b2) das Werkzeug vom teilweise bearbeiteten Rohling entfernt wird,
- d2)eine Drehung des drehbaren Trägers veranlasst wird, um den Rohling in eine andere Bearbeitungsstellung relativ zum Roboter zu bringen, und
- der Schritt b2) unter Änderung der Bearbeitungsebene mindestens einmal wiederholt wird.

6. Verfahren nach einem der Patentansprüche 1 bis 5, in dem das Werkzeug eine Fräse ist, die einen Arbeitsteil aufweist, der einen zylindrischen Teil und/oder einen halbkugelförmigen Teil (19) umfasst und Schneidorgane auf dieser oder diesen Teil(en).

7. Verfahren nach einem der Patentansprüche 1 bis 6, in dem die Drehachse (13) des drehbaren Trägers zur Gelenkachse (AX1) der beiden ersten Segmente (161, 162) des Roboters parallel ist und in einem peripheren Teil des Arbeitsraums des Roboters verläuft.

8. Verfahren nach einem der Patentansprüche 1 bis 7, in dem die Ebene des drehbaren Trägers, der den Rohling aufnimmt, in einem längs einer zur Gelenkachse (AX1) der beiden ersten Segmente (161, 162) des Roboters parallelen Achse gemessenen Maß angeordnet ist, derart dass das Maß (98) auf dieser Achse vom Zentrum (97) der Form oder des Rohlings gleich - oder nahe
- dem Maß (99) auf derselben Achse von der Gelenkachse (AX2) des zweiten und dritten Segments(162, 163) des Roboters ist.

9. Verfahren nach einem der Patentansprüche 1 bis 8, in dem die Gelenkachse (AX1) der beiden ersten Segmente (161, 162) des Roboters und die Drehachse (13) des drehbaren Trägers vertikal sind und die Ebene des drehbaren Trägers unter der Höhe der Gelenkachse(AX2) des zweiten und dritten Segments (162, 163) des Roboters in einem Abstand angeordnet ist, der der halben Höhe des Rohlings oder der Form gleich ist.

10. Verfahren nach einem der Patentansprüche 1 bis 9, in dem die Anzahl verschiedener Bearbeitungsstellungen, die gegebenenfalls der Anzahl der Drehungen des drehbaren Trägers entspricht - und der Anzahl der Arbeitsschritte b1) oder b2) - um Eins erhöht in einem Bereich zwischen 2 und 8 liegt, ib in einem Bereich zwischen 2 und 6.

11. Verfahren nach einem der Patentansprüche 4 bis 10, in dem der Drehwinkel des drehbaren Trägers 90 Grad oder 180 Grad beträgt.

12. Verfahren nach einem der Patentansprüche 5 bis 11, in dem mindestens zwei der Bearbeitungsebenen (PU1 bis PU6) rechtwinklig zueinander sind.

13. Verfahren nach einem der Patentansprüche 5 bis 12, in dem mindestens zwei der Bearbeitungsebenen (PU1 bis PU6) parallel zueinander sind.

14. Verfahren nach einem der Patentansprüche 4 bis 13, in dem für jeden Trennungs- oder "Rückzugs" -Schritt c1) oder c2) eine Rückwärtsbewegung (34) des Werkzeugs über eine Hälfte der Werkzeugbahn, die zuletzt ausgeführt wurde, veranlasst wird, gefolgt von einer Bewegung (35) des Werkzeugs auf einer Normalen zur Bearbeitungsebene (PU1 bis PU6), die dem laufenden Bearbeitungsvorgang entspricht, in Richtung einer Entfernung des Werkzeugs relativ zur Bearbeitungsebene und über eine Länge, die mindestens der halben Länge des Arbeitsteils des Werkzeugs gleich ist.

15. Vorrichtung zur Bearbeitung einer Form (15) zur Herstellung eines orthopädischen Apparates, wobei die Vorrichtung einen Träger (12) des Rohlings (10) umfasst, der um eine Drehachse (13) drehbar montiert ist, einen Roboter (16), der über mindestens fünf Drehgelenke (AX1 bis AX5) in Reihe verfügt, und eine Welle, um ein Bearbeitungswerkzeug (18) in Rotation zu versetzen, sowie eine Steuereinheit - etwa eine elektronische Steuereinheit mit Mikroprozessor -, die dafür programmiert ist, die Betätigung der Wirkglieder des Roboters zu steuern und die Drehung des drehbaren Rohlingträgers zu steuern, um die Arbeitsschritte eines Verfahrens nach einem der Patentansprüche 1 bis 14 auszuführen.

16. Vorrichtung nach Patentanspruch 15, in der sich die Gelenkachsen (AX4, AX5) des vierten, fünften und sechsten Segments (164, 165, 166) des Roboters und gegebenenfalls die Rotationsachse (X6) des Werkzeugs (18) in einem Punkt (96) treffen.

## Claims

1. A method of machining a mold (15) for fabricating an orthopedic appliance, the mold being machined using a machine (16) having at least five axes and carrying a rotary tool (18), wherein the machine is a robot that has at least five rotary joints (AX1 to AX5) in series, and wherein:
• a blank (10) of the mold is fastened to a support (12) mounted to rotate about an axis of rotation (13);
• a blank (10) of the mold is placed in succession in a plurality of distinct machining positions relative to the robot, by turning the rotary support; and
• for each of these machining positions, the blank is machined while maintaining the direction (29) of the tool substantially constant, and while moving the end of the tool along successive paths (30, 31) that extend in parallel movement planes (Pld).

2. A method according to claim 1, wherein the movement planes (Pld) are parallel to a longest axis (13) of the mold.

3. A method according to claim 1 or claim 2, wherein the movement planes (Pld) are spaced apart at a pitch close to one-fourth or one-eighth of the diameter of the cutter portion of the tool (18).

4. A method according to any one of claims 1 to 3 and including the following operations:
a1) fastening the blank of the mold on the rotary support (12);
b1) while keeping the rotary support stationary, machining the blank in part using the rotary tool while maintaining the axis (X6) (of rotation) of the tool substantially parallel to a determined direction (29) relative to the blank;
c1) moving (35) the tool away from the blank as partially machined at the end of operation b1); and
d1) selecting a second determined direction relative to the blank that is different from the first determined direction relative to the blank, and where appropriate causing the rotary support to turn through an angle of rotation that is equal to - or that is determined as a function of - the angle formed by the first and second determined directions relative to the blank; and then
repeating operation b1), while keeping the axis of rotation of the tool substantially parallel to the second determined direction relative to the blank.

5. A method according to any one of claims 1 to 4, the robot including a rotary actuator associated with each of its joints and carrying a spindle for driving the machining tool in rotation, wherein:
a2) the blank of the mold is fastened on the rotary support;
b2) while keeping the rotary support stationary so as to place the blank in one of said machining positions, driving the rotary tool and controlling the rotary actuators of the robot to move the tool so as to interfere with the blank and cause material to be removed, while maintaining the orientation of the axis of rotation of the tool substantially constant relative to a machining plane (PU1 to PU6), the actuators of the robot being controlled to move the tool along the movement planes that are substantially parallel to the axis of rotation of the rotary support, that are substantially regularly spaced apart, and that are substantially perpendicular to the machining plane;
c2) moving the tool away from the blank as partially machined at the end of operation b2); and
d2) causing the rotary support to turn so as to place the blank in another machining position relative to the robot; and
repeating operation b2) at least once, while modifying the machining plane.

6. A method according to any one of claims 1 to 5, wherein the tool is constituted by a cutter presenting an active part having a cylindrical portion and/or a hemispherical portion (19), and cutting members distributed over said portion(s).

7. A method according to any one of claims 1 to 6, wherein the axis of rotation (13) of the rotary support is parallel to the axis (AX1) of the joint between the first two segments (161, 162) of the robot and extends in a peripheral portion of the working volume of the robot.

8. A method according to any one of claims 1 to 7, wherein the plane of the rotary support receiving the blank is arranged at a measured distance along an axis parallel to the axis (AX1) of the joint between the first two segments (161, 162) of the robot, such that the position (98) along said axis of the center (97) of the mold or of the blank coincides with - or is close to - the position (99) along the same axis of the axis (AX2) of the joint between the second and third segments (162, 163) of the robot.

9. A method according to any one of claims 1 to 8, wherein the axis (AX1) of the joint between the first two segments (161, 162) of the robot and the axis of rotation (13) of the rotary support are both vertical, the plane of the rotary support being placed lower than the altitude of the axis (AX2) of the joint between the second and third segments (162, 163) of the robot, at a distance that is equal or close to half the height of the blank or of the mold.

10. A method according to any one of claims 1 to 9, wherein the number of distinct machining positions, corresponding, where appropriate, to the number of turns of the rotary support - and to the number of operations b1) or b2) - plus one, lies in the range 2 to 8, and in particular is situated in the range 2 to 6.

11. A method according to any one of claims 4 to 10, wherein the rotary support turns through an angle of 90° or 180°.

12. A method according to any one of claims 5 to 11, wherein at least two of the machining planes (PU1 to PU6) are orthogonal.

13. A method according to any one of claims 5 to 12, wherein at least two of the machining planes (PU1 to PU6) are parallel.

14. A method according to any one of claims 4 to 13, wherein for each disengagement or "retreat" operation c1) or c2), the tool is caused to move (34) backwards along half of the path most recently followed by the tool, followed by moving (35) the tool along a normal to the machining plane (PU1 to PU6) corresponding to the current machining operation, so as to move the tool and the machining plane relatively apart, and over a distance not less than half the length of the "active" part of the tool.

15. A device for machining a mold (15) for fabricating an orthopedic appliance, the device comprising a support (12) for supporting a blank (10) mounted to turn about an axis of rotation (13), a robot (16) having at least five rotary joints (AX1 to AX5) in series and carrying a spindle for driving rotation of a machining tool (18), and a control unit - such as an electronic control unit having a microprocessor - programmed to control the operation of the actuators of the robot and to control rotation of the rotary blank support in such a manner as to execute the operations of a method according to any one of claims 1 to 14.

16. A device according to claim 15, wherein the axes (AX4, AX5) of the joints between the fourth, fifth, and sixth segments (164, 165, 166) of the robot, and, where appropriate, the axis (X6) of rotation of the tool (18), coincide at a point (96).
